# EUROPEAN PATENT APPLICATION

(11) **EP 2 165 646 A1**
(43) Date of publication of application: **24.03.2010**
(21) Application number: 09170275.3
(22) Date of filing: 15.09.2009
(51) Int. Cl.: A61B 3/16

(54) **Non-contact ultrasonic tonometer**

(30) Priority: 16.09.2008 JP 2008237144
(71) Applicant: NIDEK CO., LTD, Gamagori-shi Aichi 443-0038 (JP)
(72) Inventor: Jinde, Masayuki, Gamagori-shi Aichi 443-0038 (JP); Miwa, Tetsuyuki, Gamagori-shi Aichi 443-0038 (JP); Makino, Kenichiro, Gamagori-shi Aichi 443-0038 (JP)
(74) Representative: Prüfer & Partner GbR European Patent Attorneys

(57) **Abstract**

A non-contact ultrasonic tonometer for measuring intraocular pressure of an examinee's eye (E) in a non-contact manner by use of an ultrasonic wave comprises: a probe (10) that emits an ultrasonic beam to be allowed to enter the eye and detects the ultrasonic beam reflected by the eye; and an arithmetic section (70) that determines the intraocular pressure by processing an output signal from the probe, the probe including a broadband air-coupled transducer that transmits and receives the ultrasonic beam having a broadband frequency component.

## Description

### Technical Field

The non-contact ultrasonic tonometer for measuring intraocular pressure of an examinee's eye in a non-contact manner by ultrasound (an ultrasonic wave).

### Background Art

There is proposed a non-contact intraocular pressure measurement system including a probe which emits an ultrasonic wave to be allowed to enter an eye (actually, an eyeball mode) and detects the ultrasonic wave reflected by the eye, the system being adapted to measure intraocular pressure of the eye based on a detection result of the probe (see "Development of a new non-contact intraocular pressure measurement system using a phase shift method", Masayuki JINDE and other three persons, Conference of Institute of Electrical Engineers, Sensors and Micromachines Division, Document p.93-96, 2007).

However, in the above system, a working distance of an apparatus from the eye (the eyeball model) is short and hence the apparatus may touch an examinee's eye when the intraocular pressure of the human eye is to be actually measured.

In the case of the conventional probe, propagation efficiency of an ultrasonic wave in air is low and thus a reflected wave from a cornea is apt to become small by attenuation of the ultrasonic wave in air even when the ultrasonic wave is converged by use of an acoustic lens. It is therefore difficult to precisely measure the intraocular pressure.

### Summary of Invention

### Technical Problem

The present invention has a purpose to provide a non-contact ultrasonic tonometer capable of accurately measuring intraocular pressure while ensuring a working distance of the tonometer from an examinee's eye.

### Solution to Problem

To achieve the above purpose, the present invention provides a non-contact ultrasonic tonometer for measuring intraocular pressure of an examinee's eye in a non-contact manner by use of an ultrasonic wave, the tonometer comprising: a probe that emits an ultrasonic beam to be allowed to enter the eye and detects the ultrasonic beam reflected by the eye; and an arithmetic section that determines the intraocular pressure by processing an output signal from the probe, the probe including a broadband air-coupled transducer that transmits and receives the ultrasonic beam having a broadband frequency component.
Further developments of the present invention are given in the dependent claims.

### Brief Description of Drawings

FIG. 1 is a schematic configuration view of a measurement system, optical systems, and a control system of a non-contact ultrasonic tonometer in an embodiment of the invention;
FIG. 2 is a view showing main parts of a probe;
FIGs. 3A are views showing examples of an observation screen (an anterior segment image displaying screen) displayed on a monitor; and
FIG. 4 is a view showing that a vibrator and a sensor are arranged symmetrically with respect to an optical axis of the observation optical system.

### Description of Embodiments

Towards the practical use of a non-contact ultrasonic tonometer, the present inventors had studied a probe capable of detecting a reflected wave from a cornea with high sensitivity and ensuring that a working distance of an apparatus (the probe) from an examinee's eye is at least 10 mm or longer. A main purpose thereof is to enhance measurement accuracy of intraocular pressure and ensure safety of an examinee's eye.

The inventors first tried to use a ceramic piezoelectric probe (a piezoelectric element type probe) well known as an ultrasonic probe. However, every ceramic piezoelectric probe could not avoid attenuation of ultrasonic wave in air, inevitably resulting in low detection sensitivity to the reflected wave from the cornea. Furthermore, the attenuation of ultrasonic wave in air becomes larger as the working distance of the apparatus from the examinee's eye is longer. Therefore, it was practically difficult to obtain high measurement precision while ensuring a working distance of 10 mm or longer.

Thereafter, while the inventors was looking for a probe meeting requirements of the high-sensitive detection of a reflected wave from a cornea and the ensuring of 10-mm or longer working distance, the inventors found an article titled "Non-contact detection using air-coupled ultrasonic wave" Youji EWATARI, p. 62-64 in the Dec. 2001 issue of a monthly journal titled Inspection Engineering. This article describes BAT™ (Broadband Air Transducer) of MicroAcoustic Instrument Inc. as a probe configured that air layers are provided before and behind a vibrating surface. There is described the BAT™ probe has very high propagation efficiency in air and is able to transmit and receive a broadband signal.

The inventors therefore took note that the ultrasonic wave to be transmitted and received by the BAT™ probe had excellent propagation efficiency in air. Then, they conceived the use of the BAT™ probe with a non-contact ultrasonic tonometer might remarkably enhance the propagation efficiency of an ultrasonic wave between an examinee's eye and the apparatus and also detect the reflected wave from a cornea with high sensitivity even if the working distance of the apparatus from the examinee's eye was longer than 10 mm.

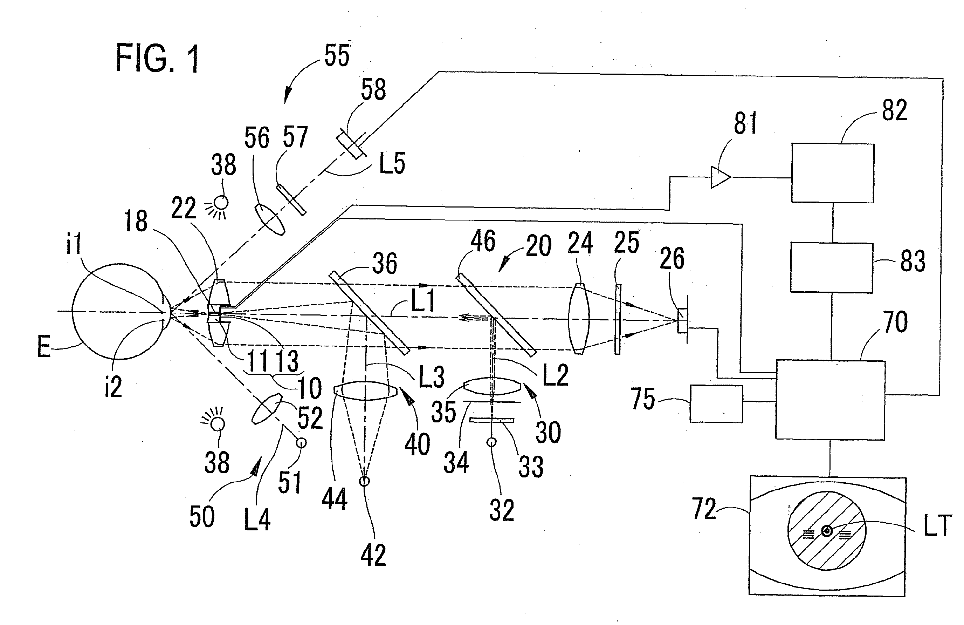

The details of a preferred embodiment of the present invention will now be given referring to the accompanying drawings. FIG. 1 is a schematic configuration view of a measurement system, optical systems, and a control system in the non-contact ultrasonic tonometer in this embodiment. The following measurement system and optical systems are contained in a housing not shown. The housing is moved three-dimensionally in a right-and-left direction (an X-direction), an up-and-down direction (a Y-direction), and a back-and-force direction (a working distance direction; a Z-direction) relative to an examinee's eye E by a well-known alignment mechanism provided in the apparatus.

A probe (a transducer) 10 placed in front of the examinee's eye E has a vibrator (an ultrasonic wave transmitting section) 11 for emitting an ultrasonic wave (an incident wave, a transmission wave) to be allowed to enter the eye E and a sensor (an ultrasonic wave receiving section) 13 for detecting an ultrasonic wave (a reflected wave, a received wave) reflected by the eye E. The probe 10 in this embodiment is adapted to operate as both the vibrator 11 and the sensor 13 under control of an arithmetic and control section 70. Alternatively, the vibrator 11 and the sensor 13 may be constituted separately.

In this embodiment, a pulse wave is used as the ultrasonic wave allowed to enter the eye E, but instead thereof a continuous wave may be used.

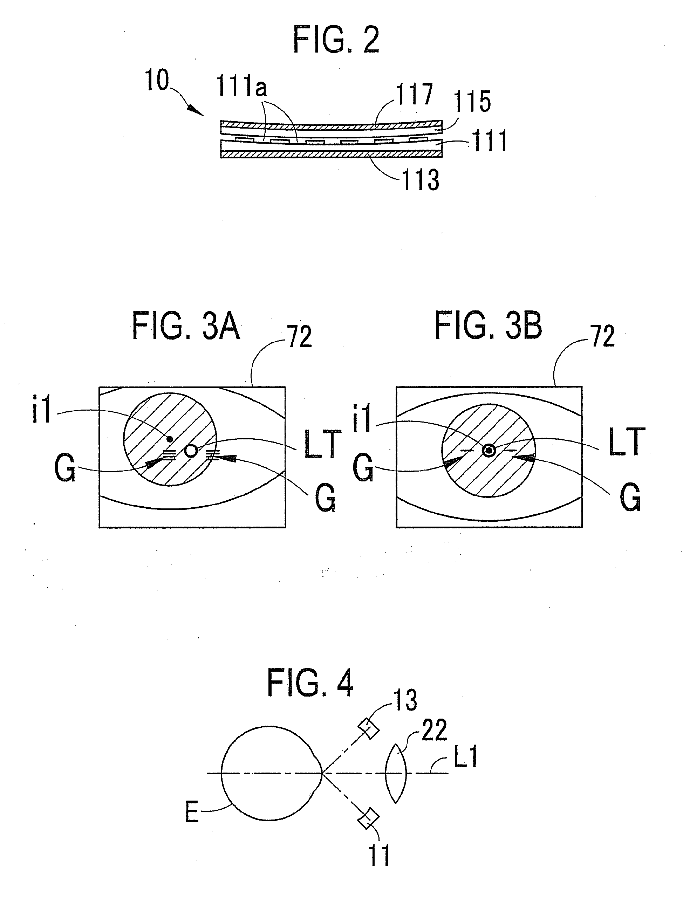

FIG. 2 is a view showing main parts of the probe 10. The probe 10 is a Broadband Air-coupled Transducer to transmit and receive an ultrasonic wave having a broadband frequency component. In this embodiment, a BAT™ probe by MicroAcoustic Instrument Inc. is used. The BAT™ probe is a condenser type (a capacitance type) sensor.

Specifically, the probe 10 includes a substrate (a back plate) 111 formed with a plurality of fine pores 111a each serving as an air pocket, a first electrode 113 placed on a lower surface of the substrate 111 (on an unroughened surface thereof), a second electrode 117 placed on an upper surface of the substrate 111 (on a roughened surface thereof), and an insulation film 115 or a dielectric film 115 (hereinafter, referred to as a "film 115") placed between the second electrode 117 and the substrate 111.

The substrate 111 may be any substrate if only one surface (an upper surface) is roughened and formed with fine air pockets.

The film 115 is made of an insulating polymer material such as polyethylene and polyimide or an insulating non-polymer material such as silicon nitride, aluminum oxide, and mica.

The substrate 111, the film 115, and the second electrode 117 each have a concavely curved shape to converge an ultrasonic beam to be irradiated to the eye E.

The probe 10 is formed, at its almost center, with an aperture 18 (e.g., a circular hole having a diameter of about 1.5 mm) through which at least one of light from a light source 32 and light from a light source 42 is allowed to pass.

When a voltage is applied between the first electrode 113 and the second electrode 117, the film 115 vibrates to generate an ultrasonic wave. The vibration of the film 115 causes an air layer between the film 115 and the substrate 111 to vibrate. Thus, the plurality of fine pores 111a function as air pockets. The air in each air pocket and the first and second electrodes 115 and 117 come into a resonant condition, thereby irradiating an ultrasonic wave with high in-air propagation efficiency to the eye E. In this case, for example, an ultrasonic wave having a broadband frequency of about 200 kHz to about 1 MHz is generated. For further details of the Broadband Air-coupled Transducer shown in FIG. 2, see US 5,287,331, WO 2003/035281 (Patent application publication No. 2005-506783), and others.

The probe 10 is connected sequentially to an amplifier 81, a frequency component analysis section 82, a frequency phase difference specifying section 83, and the arithmetic and control section 70. An electric signal corresponding to an incident wave and a reflected wave is amplified to an appropriate signal level by the amplifier 81 and subjected to frequency component analysis by the analysis section 82 to obtain a spectral distribution of the phase difference with respect to the frequency. The specifying section 83 compares the spectral distribution of the incident wave and the spectral distribution of the reflected wave to specify a phase difference θx which is a difference in phase between the incident wave and the reflected wave at respective frequencies fx. The phase difference θx at the frequency fx will vary according to intraocular pressure (strictly speaking, changes in hardness of a cornea of the examinee's eye E resulting from changes in the intraocular pressure). Accordingly, the arithmetic and control section 70 detects the phase difference θx based on an output signal of the specifying section 83 and obtains the intraocular pressure of the eye E based on the detection result. For this method, see US6,854,331 (JP2002-272743A).

Provided as the optical systems of the tonometer 100 are an observation optical system 20 for observing the anterior segment of the eye E, a fixation target projection optical system 30 for causing the eye E to hold fixation, a first alignment mark projection optical system 40 for projecting an alignment mark in the X- and Y-directions to the eye E, a second alignment mark projection optical system 50 for projecting an alignment mark in the Z-direction to the eye E, and an alignment mark detection optical system 55 for detecting the Z-direction alignment mark projected onto the eye E.

The observation optical system 20, having an optical path (hereinafter, an "observation optical path") in which the probe 10 is placed, forms an image of the anterior segment through a region surrounding the probe 10. Specifically, the observation optical system 20 includes, an objective lens 22, an imaging lens 24, a filter 25, and an image pickup device 26 and provides an optical axis (hereinafter, an "observation optical axis") L1 in which the probe 10 is placed. Thus, when the observation optical axis L1 is aligned to a predetermined portion (for example, a corneal center or a pupil center of the eye E), the probe 10 is placed in front of the eye E.

Light sources 38 which emit infrared light to illuminate the anterior segment of the eye E are disposed diagonally to the front of the eye E. The filter 25 has a property of transmitting the light from each light source 38 and the light from the light source 42 and blocking light from a light source 51.

The light from each light source 38 is projected onto the anterior segment of the eye E and then the light reflected by the anterior segment travels toward the lens 22. The light reaching the surrounding region of the probe 10 passes through the lens 22, further passes a half mirror 36 and a dichroic mirror 46, and forms an image on the image pickup device 26 by the lens 24. Specifically, the anterior segment image by the light sources 38 is formed on the image pickup device 26 through the surrounding region of the probe 10. The dichroic mirror 46 has a property of transmitting the light from each light source 38 and the light from the light source 42 and reflecting the light from the light source 32.

A signal output from the image pickup device 26 is input to the arithmetic and control section 70. The picked-up anterior segment image is displayed on a monitor 72. An imaging optical system (a lens system constituted of a plurality of lenses) including the lenses 22 and 24 is used as a light delivery member for delivering the anterior segment image to the image pickup device 26. Alternatively, a single lens may be used to deliver the anterior segment image to the image pickup device 26.

The fixation target projection optical system 30 includes at least a fixation target projection light source to project a fixation target onto the eye E to cause the eye E to view the fixation target. Specifically, the projection optical system 30 includes the light source 32, a fixation target 33, a diaphragm 34, a projection lens 35, and the dichroic mirror 46 to project the fixation target onto the eye E through the aperture 18. An optical axis L2 of the projection optical system 30 is made coaxial with the observation optical axis L1 by the dichroic mirror 46 located in the observation optical path.

Light of the fixation target 33 illuminated by visible light from the light source 32 is reduced in light diameter by the diaphragm 34, passes through the lens 35, is reflected by the dichroic mirror 46, passes through the half mirror 36, and then is projected onto a fundus of the eye E through the aperture 18. Thus, the eye E holds fixation.

The first alignment mark projection optical system 40 includes at least an alignment mark projecting light source to project an alignment mark in the X- and Y-directions onto the eye E from front. Specifically, the projection optical system 40 includes the light source 42, a projection lens 44, and the half mirror 36 to project the alignment mark (the alignment mark light) onto the eye E through the aperture 18. An optical axis L3 of the projection optical system 40 is made coaxial with the observation optical axis L1 by the half mirror 36 located in the observation optical path.

Infrared light from the light source 42 passes through the lens 44, is reflected by the half mirror 36, and then is projected onto the cornea of the eye E through the aperture 18. Light mirror-reflected by the cornea forms an image (an alignment mark image) i1 which is a virtual image (a corneal reflection image) of the light source 42.

The light of the mark image i1 travels toward the lens 22. The light reaching the surrounding region of the probe 10 passes through the lens 22, the half mirror 36, and the dichroic mirror 46, and forms an image on the image pickup device 26 by the lens 24. In other word, the mark image i1 by the light source 42 is formed on the image pickup device 26 through the surrounding region of the probe 10. When the eye E moves in the X- and Y-directions, an image forming position of the mark image i1 also moves on the image pickup device 26. Based on a detection signal of the image pickup device 26, the arithmetic and control section 70 can detect an alignment state of the apparatus (the probe 10) in the X- and Y-directions with respect to the eye E.

The second alignment mark projection optical system 50 includes at least an alignment mark projecting light source to project an alignment mark in the Z-direction onto the eye E from an oblique direction. Specifically, the projection optical system 50 includes the light source 51 and a projection lens 52 to project the alignment mark (the alignment mark light) onto the eye E. An optical axis L4 of the projection optical system 50 intersects with the observation optical axis L1 at a predetermined angle.

Infrared light from the light source 51 passes through the lens 52, is substantially collimated, and then is projected onto the cornea of the eye E. The light mirror-reflected by the cornea forms an image (an alignment mark image) i2 which is a virtual image (a corneal reflection image) of the light source 51.

The alignment mark detection optical system 55 includes a photo-receiving lens 56, a filter 57, and a position sensitive device 58 (e.g., a line CCD) to detect the alignment mark image formed by the projection optical system 50. The filter 57 has a property of transmitting the light from the light source 51 and blocking the light from the light source 38 and the light from the light source 42. An optical axis L5 of the detection optical system 55 is symmetrical to the optical axis L4 of the projection optical system 50 with respect to the observation optical axis L1. The optical axis L5 intersects with the optical axis L4 at a point on the optical axis L1.

The mark image i2 by the light source 51 is formed on the position sensitive device 58 by the lens 56. When the eye E moves in the Z-direction, an image forming position of the mark image i2 moves on the position sensitive device 58. Based on a detection signal of the position sensitive device 58, the arithmetic and control section 70 can detect an alignment state of the apparatus (the probe 10) in the Z-direction with respect to the eye.

The arithmetic and control section 70 is coupled to the monitor 72, the specifying section 83, the light sources 32, 38, 42, and 51, the image pickup device 26, the position sensitive device 58, a memory 75 serving as a storage section, and others. The arithmetic and control section 70 performs control of the entire apparatus, calculation of measured values, and so on.

The memory 75 stores a table showing a correlation between the phase difference θx at the frequency fx and an intraocular pressure value. The arithmetic and control section 70 retrieves an intraocular pressure value corresponding to the detected phase difference θx from the memory 75 based on the output signal of the specifying section 83 and displays the retrieved intraocular pressure value on the monitor 72.

The correlation between the phase difference θx and the intraocular pressure value can be set by experimentally determining in advance a correlation between phase differences θx obtained by the present apparatus and intraocular pressure values measured by a Goldmann tonometer. The memory 75 stores a program for measuring intraocular pressure by use of the probe 10, a program for controlling the entire apparatus, and so on.

Operations of the apparatus having the above configuration are explained below. Firstly, an examiner makes alignment of the apparatus (the housing containing the measurement system and the optical systems) with the examinee's eye E by manipulating a joystick not shown while viewing the monitor 72. At that time, the arithmetic and control section 70 displays a composite image including the anterior segment image picked up by the image pickup device 26 and a reticle LT and an indicator G for alignment on the monitor 72 as shown in FIGs. 3A and 3B. The arithmetic and control section 70 controls the display of the indicator G based on the information on the alignment state in the Z-direction obtained from the detection signal from the position sensitive device 58.

The examiner manipulates the joystick so that the mark image i1 displayed on the monitor 72 moves into the reticle LT and the indicator G appears in the form representing alignment completion (see FIG. 3B). When the alignment is completed in each direction and a measurement start switch not shown is pressed by the examiner, the arithmetic and control section 70 generates a measurement-start trigger signal to start the intraocular pressure measurement.

Upon generation of the measurement-start trigger signal, the arithmetic and control section 70 causes the probe 10 to emit the ultrasonic wave allowed to enter the eye E and detect the ultrasonic wave reflected from the eye E. The arithmetic and control section 70 calculates an intraocular pressure value of the eye E based on the output signal of the specifying section 83 and displays a result thereof on the monitor 82.

Since the broadband air-coupled transducer is used as the probe 10 as above, the propagation efficiency of an ultrasonic wave in air can be remarkably enhanced. Even if the working distance of the apparatus from the eye E is made longer, the reflected wave from the cornea can be detected with high sensitivity. Specifically, in the apparatus in this embodiment, a proper working distance of the apparatus from the eye E in the intraocular pressure measurement can be set to be longer than at least 10 mm. The setting of the proper working distance of the apparatus from the eye E to be longer than 10 mm is to avoid eyelashes from touching a part of the apparatus when the eye E blinks or avoid other disadvantages.

Furthermore, in the case where the emitting surface of the probe 10 is formed in a concave shape as above to converge the ultrasonic beam, it is preferable to design the concave surface so that an irradiating area of the ultrasonic beam falls within an eyelid opening area of the eye E (e.g., a curvature radius of the concave surface of the emitting surface of the probe 10 is set equal to the proper working distance of the apparatus from the eye E). This makes it possible to restrict the irradiating area of the ultrasonic beam to a cornea area of the eye E. Accordingly, it is possible to prevent the ultrasonic beam reflected from the eyelid or the like of the eye E from becoming noise whereby measurement accuracy is decreased.

In the aforementioned description, the emitting surface of the probe 10 is formed in the concave shape but may be formed in a planer shape. In this case, the emitting surface of the probe 10 is preferably designed so as to irradiate the ultrasonic beam to only the cornea of the eye E. For instance, it is conceivable to make an emitting area of the probe 10 smaller than the eyelid opening area of the eye E.

Even if the emitting surface of the probe 10 has a planar shape, it can converge the ultrasonic beam. In this case, it is conceivable to design the second electrode 117 in such a configuration (a so-called acoustic Fresnel zone plate) that part of the second electrode 117 is removed in ring shape to form multiple concentric ring patterns on the emitting surface. In this case, the ultrasonic waves generated from the ring-shaped electrodes 117 interfere with one another and thus the ultrasonic beam are converged.

The probe 10 in this embodiment can reduce the influence of a spherical wave generated from the probe 10 as compared with the conventional ceramic piezoelectric probe. Accordingly, it is possible to easily prevent the ultrasonic beam to be irradiated to the eye E from being irradiated to a portion other than the cornea.

As mentioned above, the aperture 18 is provided at the almost center of the probe 10. Therefore, the fixation target and the alignment mark can be projected onto the eye E even when the probe 10 is placed in front of the eye E. In this case, since the broadband air-coupled transducer as mentioned above is used, an air pocket is formed in every pore 111a formed in the substrate 111, causing resonance of air in each air pocket. Accordingly, even when the aperture 18 is provided at the almost center of the probe 10, a stable ultrasonic wave can be generated. On the other hand, in the case of the conventional ceramic piezoelectric probe, the frequency characteristics of the probe are apt to vary due to the aperture formed therein. Thus, the frequency characteristics of the ultrasonic wave generated by the probe can become unstable.

In the above explanation, the intraocular pressure of the eye E is determined based on the difference in phase between the incident wave to the eye E and the reflected wave from the eye E. The invention is not limited thereto and the intraocular pressure of the eye may be determined by detecting acoustic intensity of the reflected wave from the eye E. In this case, the difference in acoustic impedance between the cornea and the air is larger as the intraocular pressure is higher and therefore the acoustic intensity is detected to be high. To the contrary, the difference in acoustic impedance between the cornea and the air is smaller as the intraocular pressure is lower and therefore the acoustic intensity is detected to be low. For instance, it is conceivable to calculate acoustic impedance of the cornea based on acoustic intensity of the reflected wave from the probe 10, and calculate the intraocular pressure of the eye E based on the calculated acoustic impedance. In this case, it is only necessary to determine in advance a relationship between the acoustic impedance (or the acoustic intensity) and the intraocular pressure of the eye E.

In the above explanation, the probe 10 that transmits and receives ultrasonic wave is placed in front of the eye E. As an alternative, as shown in FIG. 4, the vibrator (an air-coupled ultrasonic transmitting probe) 11 for allowing the ultrasonic wave to enter the eye E and the sensor (an air-coupled ultrasonic receiving probe) 13 for detecting the ultrasonic wave reflected by the eye E may be placed symmetric with respect to the observation optical axis L1.

While the presently preferred embodiment of the present invention has been shown and described, it is to be understood that this disclosure is for the purpose of illustration and that various changes and modifications may be made without departing from the scope of the invention as set forth in the appended claims.

## Claims

**1.**
A non-contact ultrasonic tonometer for measuring intraocular pressure of an examinee's eye (E) in a non-contact manner by use of an ultrasonic wave, the tonometer comprising:
a probe (10) that emits an ultrasonic beam to be allowed to enter the eye and detects the ultrasonic beam reflected by the eye; and
an arithmetic section (70) that determines the intraocular pressure by processing an output signal from the probe,
the probe including a broadband air-coupled transducer that transmits and receives the ultrasonic beam having a broadband frequency component.

**2.**
The tonometer according to claim 1, wherein
the probe has an emission diameter or an emitting surface shape determined to irradiate the ultrasonic beam to only a cornea of the eye.

**3.**
The tonometer according to claim 2, wherein
the emission diameter or the emitting surface shape of the probe is determined so that an irradiating area of the ultrasonic beam at a proper working distance longer than at least 10 mm between the eye and the tonometer falls within a cornea area of the eye.

**4.**
The tonometer according to any of claims 1 to 3, wherein
the probe has an emitting surface formed in a concave shape or an emitting surface formed as a Fresnel zone plate to converge the irradiated ultrasonic beam.

**5.**
The tonometer according to any of claims 1 to 4, wherein
the probe includes: a substrate (111) having one surface that is roughened; a first electrode (113) placed on the other unroughened surface of the substrate; a second electrode (117) placed on the roughened surface of the substrate; and an insulating film (115) or a dielectric film (115) placed between the second electrode and the substrate,
vibration of the film caused when a voltage is applied between the first and second electrodes causes an air layer formed between the film and the substrate to vibrate and generate the ultrasonic wave.

**6.**
The tonometer according to any of claims 1 to 5 further comprising:
a fixation target projection optical system (30) provided with a light source (32) for fixation target projection to project a fixation target onto the eye,
the probe having an aperture (18) allowing a fixation target beam from the light source to pass through.

**7.**
The tonometer according to any of claims 1 to 6 further comprising:
an alignment mark projection optical system (40) provided with a light source (42) for alignment mark projection to project an alignment mark onto the eye,
the probe having an aperture (18) allowing an alignment mark beam from the light source to pass through.

**8.**
The tonometer according to any of claims 1 to 7 further comprising:
an anterior segment observation optical system (20) for observing an anterior segment of the eye,
the probe being placed in an optical path of the anterior segment observation optical system.
